Europäisches Patentamt

European Patent Office

Office européen des brevets

(11) Publication number: 0 134 773
A1

# EUROPEAN PATENT APPLICATION

(21) Application number: 84870112.4

(22) Date of filing: 07.08.84

(51) Int. Cl.⁴: **C 12 N 15/00**

(30) Priority: 08.08.83 US 520668
02.03.84 US 584657

(43) Date of publication of application:
20.03.85 Bulletin 85/12

(84) Designated Contracting States:
AT BE CH DE FR GB IT LI LU NL SE

(71) Applicant: SMITHKLINE BECKMAN CORPORATION
P.O. Box 7929 1 Franklin Plaza
Philadelphia Pennsylvania 19101(US)

(72) Inventor: Butt, Tauseef Rashid
431 Beech Tree Lane
Wayne Pennsylvania 19087(US)

(74) Representative: Tasset, Gérard
SMITHKLINE - RIT rue de l'Institut, 89
B-1330 Rixensart(BE)

(54) Yeast copper-metallothionein gene.

(57) A copper-inducible *Sau*3A-*Sau*3A fragment of yeast chromosomal DNA carrying the gene for Yeast Copper-Metallothionein (Cu-Mt) can be isolated by hybridization with mRNA from copper-resistant cells and used to produce Cu-Mt.

EP 0 134 773 A1

-1-

## Title

Yeast Copper-Metallothionein Gene

## Field of the Invention

This invention relates to cloning of a gene coding for Yeast Copper-Metallothionein.

## Background Information

Metallothioneins are low molecular weight, cysteine-rich proteins which bind transition metals. They are present in vertebrates, invertebrates, plants and lower eukaryotes and are largely evolutionarily conserved. Metallothioneins, metallothionein-like proteins and metallothionein-producing microorganisms, can be used to chelate transition metals, such as metals found in industrial waste streams and mining and mineral processing fluids. In addition, they can be used in the treatment of metals poisoning, such as by passing a subject's plasma through columns having a metallothionein bound thereto.

Fogel et al., Proc. Natl. Acad. Sci. U.S.A. 79:5342-5346 (1982), report isolation and cloning of yeast DNA fragments that confer copper resistance (Cu$^r$) on otherwise copper sensitive (Cu$^s$) cells. Each cloned fragment is composed of tandemly iterated 1.95 kb DNA segments. The smallest fragment found to confer copper resistance is a 1.25 kb Sau3A-Sau3A fragment having an XbaI site and a KpnI site.

Butt, J. Cell. Biochem., Sup 7A, 147 (1983) report construction of a DNA library from copper-resistant yeast in YEp13 and transformation of Cu$^s$ cells to Cu$^r$ cells by certain of the plasmids.

## SUMMARY OF THE INVENTION

The invention resides in discovery of a gene from the yeast, Saccharomyces cerevisiae, which can code for Yeast Copper-Metallothionein (Cu-Mt), a cysteine- and serine-rich, metallothionein-like protein, and its promoter region. In particular the invention is an isolated structural gene derived from yeast which can code for Cu-Mt, and mutants and genetically engineered derivatives thereof.

Another aspect of the invention is an isolated gene expression unit comprising the Cu-Mt promoter and the isolated structural gene and mutants and genetically engineered derivatives thereof.

Another aspect of the invention is an isolated gene promoter region comprising the Yeast Copper-Metallothionein promoter anu mutants and geneticaily engineered derivatives thereof.

Yet another aspect of the invention is recombinant DNA molecules comprising the described DNA fragments, exemplified by plasmids YEp1, YEp2, YRp20, YEp29 and YEp36.

## DESCRIPTION OF THE FIGURE

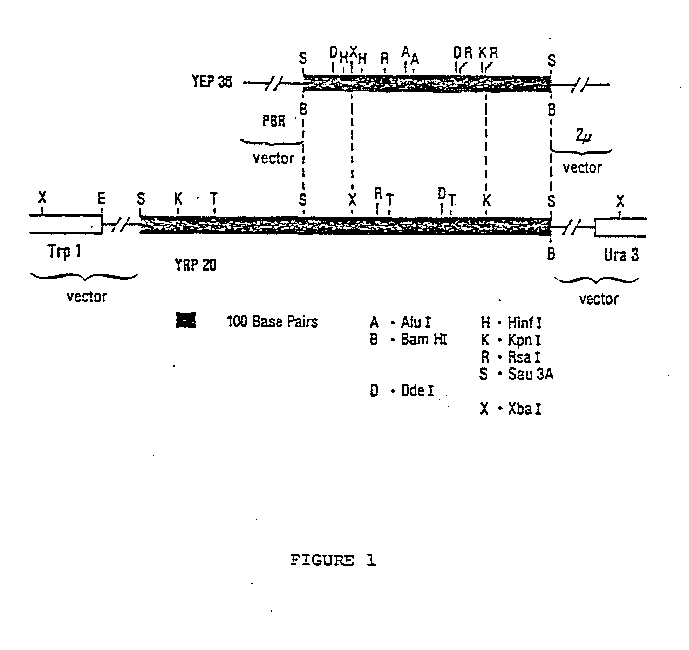

Fig. 1 is a restriction endonuclease cleavage map of Yeast DNA fragments carrying a gene expression unit of Cu-Mt.

## DETAILED DESCRIPTION OF THE INVENTION

The DNA fragments of the invention are isolatable from copper resistant strains of Saccharomyces cerevisiae as double stranded DNA (dsDNA) molecules. The invention includes said dsDNA molecules as well as equivalents thereof. Such equivalents include, but are not limited to, single stranded DNA having the same or substantially the same nucleotide sequence as one strand of the dsDNA, messenger RNA (mRNA) transcripts and DNA and RNA molecules having different codons coding for the same or substantially the same amino acids. By "gene expression unit" is meant a structural gene and regulatory sequences required for expression of the structural gene.

Copper resistant ($Cu^r$) yeast strains (resistant to 1.0 mM $CuSO_4$ in synthetic medium) can be made from laboratory yeast strains, most of which, if not all, are copper sensitive ($Cu^s$) (sensitive to 0.5 mM $CuSO_4$ in synthetic medium), by growing up such yeast cells in increasing concentrations of $CuSO_4$ and selecting $Cu^r$ survivors.

The DNA fragments of the invention can be isolated from $Cu^r$ yeast strains by restricting $Cu^r$ chromosomal DNA and assaying resulting fragments for hybridization to RNA transcripts from $Cu^r$ strains but not significantly to RNA transcripts from $Cu^s$ strains. Alternatively, the fragments can be assayed for hybridization to complementary DNA probes prepared by reverse transcription of mRNA transcripts from $Cu^r$ and $Cu^s$ strains.

The fragments can be cloned in vectors, including cosmid, plasmid and phage vectors, in yeast and other eukaryotes and in prokaryotes, by known techniques. The Cu-Mt structural gene can be expresed from its natural promoter or from a heterologous promoter.

Exemplary of vectors useful for cloning the fragments in yeast, and for expressing the structural gene from its natural promoter, are yeast plasmids YEp13 (ATCC 37115) and YRp17 (ATCC 37078), both of which are widely available. YEp13 is a pBR322 derivative which carries the 2u plasmid origin of replication and the yeast leucine gene for selection purposes.  See Broach et al., Gene 8:121-133 (1979).  YRp17 contains the Trp replicator and the yeast Ura3 gene for selection purposes and the pBR322 origin of replication.  Other vectors are, of course, also useful.

In particular embodiments, four Sau3A-Sau3A fragments of chromosomal DNA from Cu^r SC-3 strains [ura3 trp1 his3 gal2 gal10] of approximately 1350 bp were cloned into YEp13.  One other Sau3A fragment was cloned into YRp17.  The four YEp13 derivatives are identified as YEp1, YEp2, YEp29 and YEp36; the YRp17 derivative is identified as YRp20.

The YEp36 insert has been partially sequenced as follows:

|  | 10 | 20 | 30 |
|---|---|---|---|
| (5') | GGATCCCATT | ACCGACATTT | GGGCGCTATA |
|  | 40 | 50 | 60 |
|  | CGTGCATATG | TTCATGTATG | TATCTGTATT |
|  | 70 | 80 | 90 |
|  | TAAAACACTT | TTGTATTATT | TTTCCTCATA |
|  | 100 | 110 | 120 |
|  | TATGTGTATA | GGTTTATACG | GATGATTTAA |
|  | 130 | 140 | 150 |
|  | TTATTACTTC | ACCACCCTTT | ATTTCAGGCT |
|  | 160 | 170 | 180 |
|  | GATATCTTAG | CCTTGTTACT | AGTTAGAAAA |
|  | 190 | 200 | 210 |
|  | AGACATTTTT | GCTGTCAGTC | ACTGTCAAGA |
|  | 220 | 230 | 240 |
|  | GATTCTTTTG | CTGGCATTTC | TTCTAGAAGC |
|  | 250 | 260 | 270 |
|  | AAAAAGAGCG | ATGCGTCTTT | TCCGCTGAAC |
|  | 280 | 290 | 300 |
|  | CGTTCCAGCA | AAAAAGACTA | CCAACGCAAT |

-5-

0134773

| 310 | 320 | 330 |
|---|---|---|
| ATGGATTGTC | AGAATCATAT | AAAAGAGAAG |

| 340 | 350 | 360 |
|---|---|---|
| CAAATAACTC | CTTGTCTTGT | ATCAATTGCA |

| 370 | 380 | 390 |
|---|---|---|
| TTATAATATC | TTCTTGTTAG | TGCAATATCA |

| 400 | 410 | 420 |
|---|---|---|
| TATAGAAGTC | ATCGAAATAG | ATATTAAGAA |

| 430 | 440 | 450 |
|---|---|---|
| AAACAAACTG | TACAATCAAT | CAATCAATCA |

| 460 | 470 | 480 |
|---|---|---|
| TCACATAAAA | TGTTCAGCGA | ATTAATTAAC |

| 490 | 500 | 510 |
|---|---|---|
| TTCCAAAATG | AAGGTCATGA | GTGCCAATGC |

| 520 | 530 | 540 |
|---|---|---|
| CAATGTGGTA | GCTGCAAAAA | TAATGAACAA |

| 550 | 560 | 570 |
|---|---|---|
| TGCCAAAAAT | CATGTAGCTG | TCCAACGGGG |

| 580 | 590 | 600 |
|---|---|---|
| TGTAACAGCG | ACGACAAATG | CCCTTGCGGT |

| 610 | 620 | 630 |
|---|---|---|
| AACAAGTCTG | AAGAAACCAA | GAAGTCATGC |

| 640 | 650 | 660 |
|---|---|---|
| TGCTCTGGGA | AATGAAACGA | ATAGTCTTTA |

| 670 | 680 | 690 |
|---|---|---|
| ATATATTCAT | CTAACTATTT | GCTGTTTTTA |

| 700 | 710 | 720 |
|---|---|---|
| ATTTTTAAAA | GGAGAAGGAA | GTTTAATCGA |

| 730 | 740 | 750 |
|---|---|---|
| CGATTCTACT | CAGTTTGAGT | ACACTTATGT |

| 760 | 770 | 780 |
|---|---|---|
| ATTTTGTTTA | GATACTTTGT | TAATTTATAG |

| 790 | 800 | 810 |
|---|---|---|
| GTATACGTTA | ATAATTAAGA | AAAGGAAATA |

0134773

|  | 820 | | 830 | | 840 |
|---|---|---|---|---|---|
|  | AAGTATCTCC | | ATATGTCGCC | | CCAAGAATAA |
|  | 850 | | 860 | | 870 |
|  | AATATTATTA | | GCAAATTCTA | | GTTTGCCTAA |
|  | 880 | | 890 | | 900 |
|  | CTTACAACTC | | TGTATAGAAT | | CCCCAGATTT |
|  | 910 | | 920 | | 930 |
|  | CGGATAAAAA | | AAAAAAAAAA | | AGCTATTCAT |

940
GGTACC (3')

Based on the sequence, it appears that the Goldberg-Hogness Box approximately comprises base pairs 361 to 368. The initiation codon begins at base pair 460. The single reading frame codes for a 60 amino acid protein which contains 12 cysteine and 8 serine residues. Results of S1 mapping experiments indicate that transcription initiates at about base pair 401. Based on an estimated size of mature (poly-A) Cu-Mt mRNA of about 540 bases, it appears that the 3' end of the Cu-Mt sequence is in the region of base pair 835, the poly-A tail comprising approximately 100 residues.

The termination codon begins at base pair 643. The promoter region appears to begin about 10 bp upstream (5') of a Xbal restriction endonuclease site at bp 232.

The inserts in YEp29 and YEp36 appear to be identical but in different orientation. The inserts in YEp1 and YEp2 appear to be similar to YEp29 and YEp36 although YEp1 and 2 are not cleaved by BamHI. All of the five clones bear close homology in a XbaI-KpnI fragment which contains approximately 705 bp.

Restriction endonuclease mapping of the YEp36 and YRp20 inserts is shown in Figure 1. Based on the sequence data, above, it appears that the sites in the YEp36 insert are 3' to the following indicated bases:

| BamHI | 1 | DdeI | 157 |
|-------|---|------|-----|
| Sau3A | 1 | HinfI | 211,312 |
| AluI | 521, 557 | RsaI | 431 |
| XbaI | 232 | KpnI | 936 |

BbvI was also found to cut the insert. These and other restriction sites can be located by the sequence data. For example, the sequence shows BbvI sites 3' to bases 509, 544 and 617. The YRp20 insert is about 2,250 bp. As shown in Fig. 1, it has in common with the YEp36 insert the Sau3A (1), XbaI, KpnI, Sau3A (1350) and BamHI (1350) restriction sites. The region of the YRp20 insert upstream of the Sau3A (1) site contains a TaqI site about 475 bp upstream of the Sau3A (1) site, a KpnI site about 690 bp upstream (5') of the Sau3A (1) site and a Sau3A site about 905 bp upstream (5') of the Sau3A (1) site.

It is to be understood that the above sequence and restriction site data are substantially accurate, that is, actual sequences and restriction sites may differ to the extent that the activity of the molecules or of portions thereof are not materially altered.

The upstream regulatory regions, the structural gene, and any other fragment of the Sau3A-Sau3A inserts can be isolated from yeast chromosomal DNA or other yeast DNA sequence comprising such fragment by known techniques. These include, for example, restricting the yeast DNA upstream and downstream of the fragment and, if necessary, digesting the restriction fragment with a nuclease. Deoxynucleotides can be added or deleted to place the fragment into correct reading frame.

A convenient site upstream of the structural gene in the YEp36 insert is the RsaI site at bp 431. Downstream of the structural gene, the KpnI site can be used. Other convenient sites are present and can be identified from the sequence of the insert.

By way of further example, in the YEp36 insert, the Sau3A-KpnI fragment and the DdeI-KpnI fragment comprise the entire gene expression unit; the Sau3A-KpnI fragment, the XbaI-KpnI fragment and the RsaI-KpnI fragment all comprise the entire structural gene; the Sau3A-KpnI fragment, the Sau3A-RsaI fragment (about 431 bp) and the DdeI-RsaI fragment (about 274 bp) all comprise the Cu-Mt promoter region. Such and other fragments can be fused to other DNA molecules, such as cloning and expression vectors, to prepare recombinant DNA molecules, by known techniques. Substantially similar fragments can be isolated from the other inserts, although, as noted previously, the YRp20 insert apparently lacks the DdeI (157) site and the YEp1 and 29 inserts are not cleaved by BamHI.

The Cu-Mt structural gene can be expressed from its own or another (heterologous) promoter region. Cu-Mt so produced can be used to extract metal ions from waste streams and mining fluids, including leachates. It can also be used in the treatment of metals poisoning such as by passing a subject's plasma through a Cu-Mt-containing adsorbent. Such uses can be readily practiced by techniques known in the art.

The Cu-Mt regulatory region, including the promoter region, can be used to express the Cu-Mt or heterologous structural genes. It is advantageous because it is a strong, amplifiable promoter which can be regulated by copper ions.

Mutants and genetically engineered derivatives of the various DNA molecules of the invention can be prepared by known techniques including, for example, such conventional techniques as chemical or irradiation mutagenesis as well as recombinant DNA techniques such as altering the DNA sequences by adding, deleting or substituting bases.

In the following examples of the invention, which are illustrative, and not limiting, all temperatures are in degrees Celsius. All materials used, including the restriction enzymes, are commercially available and were used as per vendors' instructions.

The yeast strain, S. cerevisiae SC-3 (provided by Dr. William Bergman, University of Maryland, Baltimore County), was grown in synthetic media which contained 0.67% yeast nitrogen base, 2% glucose and appropriate amino acids and pyrimidines, substantially as described by Sherman et al., "Methods in Yeast Genetics Laboratory Manual," Cold Spring Harbor Laboratory, New York (1981). Transformations were carried out substantially by the procedure of Tschumper et al., Gene 10:157-166 (1980).

EXAMPLE 1

Preparation of $Cu^r$ Strains

Yeast cultures were grown in 0.5 mM $CuSO_4$ for several generations in synthetic media. This concentration of $CuSO_4$ is sublethal to the SC-3 strain and 50% of the cells survived. The copper sulfate concentration was increased to 1.0 mM and growth was allowed for several generations. Most of the cells in the culture died at 1.0 mM $CuSO_4$; the survivors (0.1%), which adapted to 1 mM $CuSO_4$, were plated on synthetic medium plates containing 1 mM $CuSO_4$. The $Cu^r$ strains were maintained in the presence of 1 mM $CuSO_4$. Similarly, the $Cu^s$ strains were selected from the parent culture by replica plating to synthetic medium plates containing 0.1 mM, 0.2 mM, 0.3 mM and 0.5 mM $CuSO_4$. The strains whose growth was retarded at 0.2 mM $CuSO_4$ and which did not grow at all above 0.3 mM were selected and designated as $Cu^s$.

Rapidly growing single colonies were isolated and the Cu-Mt content of the strains were estimated by $^{35}$S-cysteine incorporation and subsequent analysis of the protein by polyacrylamide gel electrophoresis as follows.

The appropriate yeast strain was grown in synthetic medium to mid-log phase ($1 \times 10^7$ cells/ml) in the presence or absence of $CuSO_4$. Prior to addition of $^{35}$S-cysteine (790 Ci/mmol), the cells were washed with distilled water, suspended in fresh media (without copper) and pulsed for 15-30 min with $^{35}$S-cysteine (50 uCi/30 ml culture). The cells were centrifuged at 3000xg for 5 min and pellets were washed with cold distilled water. The pellets were then suspended in 0.25 ml (in 1.5 ml microcentrifuge tube) 1 M sorbitol, 10 mM tris-HCl (pH 8), 1 mM ethylenediametetraacetate (EDTA), 1 mM phenyl methyl sulfonyl fluoride (PMSF) and 10 mM mercaptoethanol. Spheroplasts were prepared by adding 20 uL of Zymolyase 60,000 hydroxylase solution (Miles Laboboratories, Elkhardt, IN) (2 mg/ml) for 45 min at 37°. A nonionic detergent, NP 40, was added to a final concentration of 0.4% and the suspension was kept at 4° for 30 min after brief vortexing. The suspension was centrifuged in a microfuge for 5 min. The supernatant was treated with 0.3 M iodoacetic acid (1/10 of volume, pH 8) for 45 min and made 0.1% sodium dodecyl sulfate (SDS) and 8 M urea by the addition of solid urea crystals. Protein concentrations were estimated by the Bio-Rad dye method. Proteins were analyzed by 8 M urea polyacrylamide gel electrophoresis, as described by LeStourgeon et al., <u>Arch. Biochem. Biophys.</u> <u>155</u>:144-158 (1973), except that SDS was omitted from the gel and the buffer. 100 ug of protein was analyzed per gel slot. The gel was stained with Coomassie brilliant blue. The gel was fixed with Autoflour (National Diagnostics) for one hour, dried under vaccuum and autoradiographed. Cu$^r$ strains produced large amounts of Cu-Mt. Cu$^s$ strains produced relatively very small amounts.

EXAMPLE 2

<u>Isolation and Cloning of Cu-Mt Gene</u>

A. Complementary DNA (cDNA) probes were prepared as follows. Yeast strains, (grown in 1.0 mM $CuSO_4$) $Cu^r$ and $Cu^s$ (grown in absence of $CuSO_4$) were grown in synthetic media to a density of $2 \times 10^7$ cells/ml at $30^o$. Immediately before harvesting, the cultures were made 100 ug/ml in cycloheximide and then transferred to cold, centrifuge bottles and centrifuged at 2000xg for 5 min. Cell pellets were suspended in (7.5 ml/liter pellet) 8 M guanidine hydrochloride, 20 mM sodium acetate and 1 mM dithiothreitol. All the procedures were performed at $4^o$ in glassware which was rinsed with 0.1% diethyl pyrocarbonate (DEPT) and baked at $250^o$ overnight. Ten g of glass beads were added to the suspension and the cells were sheared in a Virtis bead beater for three 15 sec pulses. The suspension was filtered and the filtrate was centrifuged for 10 min at 5000xg. Poly A-containing RNA was purified from supernatant by the procedure of Chirgirin et al., Biochem. 18:5294-5299 (1979). Poly A-containing RNA was labelled with $^{32}$P-dCTP (7000 Ci/mmol) by synthesizing cDNA with reverse transcriptase, as described by Wickens et al., J. Biol. Chem. 253:2483-2495 (1978). Radioactive cDNA was used as a probe to isolate the copper-induced genes of yeast. On average, the specific activity of the probes was $1 \times 10^8$ cpm/ug poly-A RNA.

B. Yeast chromosomal DNA fragments were prepared as follows. Yeast DNA was isolated from $Cu^r$ SC-3 strains as described by Sherman et al., Cold Spring Harbor Laboratory, New York, 1981. Three mg of the DNA were digested to completion with Sau3A (1000 units, 4 hr, $37^o$). The fragments were fractionated into two sets of 60 fractions by preparative 1% agarose gel electrophoresis.

C. Cu-Mt-containing fragments were isolated and cloned as follows. After electrophoresis, the fractions were transferred to a nitrocellulose filter. The filter was cut in half to separate the two sets of fractions. Each filter was hybridized to a $^{32}$P-labelled cDNA probe from the Cu$^r$ or Cu$^s$ yeast strains. One fraction, in the range of 1350 bp, which hydridized with the Cu$^r$ cDNA but not the Cu$^s$ DNA was cloned into BamHI sites in YEp13 and YRp17.

Of approximately 500 clones, eight hybridized with the duplicate nitrocellulose filter. The procedure is described by Maniatis, "Molecular Cloning. A Laboratory Manual," Cold Spring Harbor Laboratory, New York 1982.

Five of the eight clones, YEp1, YEp2, YEp29, YEp36 and YRp20, were subsequently confirmed to carry the Sau3A-fragment containing the Cu-Mt gene expression unit by RNA hydridization, restriction mapping and transformation of Cu$^s$ cells.

## EXAMPLE 3
### Transformation of Cu$^s$ to Cu$^r$ Phenotype

Copper-sensitive yeast strains were transformed with YEp13, YEp36 and YRp20. The transformants were grown for three days in normal plates and then transferred to plates containing 1 mM CuSO$_4$ and grown for another three days. Cu$^s$ strains transformed with YEp13 (no DNA insert) did not grow at all in 1 mM CuSO$_4$, while the transformants of YEp36 and YRp20 grew rapidly in 1 mM CuSO$_4$.

Protein analysis of YEp36 transformants grown in the presence of 1 mM CuSO$_4$ indicated that they produced abundant quantities of mRNA which cross hydridized with the DNA insert from YEp36 and also induced expression of large quantities of Cu-Mt.

## EXAMPLE 4
### Expression of Heterologous Gene from Cu-Mt Promoter

Plasmid YEp36 was digested with Sau3A and RsaI to release a 431 bp fragment carrying the Cu-Mt promoter region. The Sau3A-RsaI fragment was cloned upstream of the GalK structural gene from E. coli in pYSK7-54. The plasmid, pYSK7-54, has BamHI and HpaI sites upstream of the GalK gene, which sites were used for insertion of the Cu-Mt promoter. The plasmid also has the pBR322 origin of replication for replication in E. coli and the arsl and cen3 regions for replication in yeast.

The resultant construct was used to transform a GalK⁻ strain of S. cerevisiae. Transformants showed high galactokinase activity in the presence of sub-lethal amounts of $CuSO_4$.

While the preferred embodiments of the invention are fully disclosed by the above, it is to be understood that the invention is not limited to the precise constructions disclosed herein but rather includes all embodiments and modifications coming within the scope of the following claims.

Claims for the Contracting States
BE, CH, DE, FR, GB, IT, LI, LU, NL, SE **0134773**

I claim:

1. An isolated structural gene derived from yeast which can code for Yeast Copper-Metallothionein, and mutants and genetically engineered derivatives thereof.

2. The gene of claim 1 which is present in a Sau3A-Sau3A fragment of chromosomal DNA in a copper-resistant (1 mM CuSO$_4$) mutant.

3. The gene of claim 2 which is present in a Sau3A-KpnI fragment of the chromosomal DNA.

4. The gene of claim 2 which is present in a XbaI-KpnI fragment of the chromosomal DNA.

5. The gene of claim 2 which is present in a RsaI-KpnI fragment of the chromosomal DNA.

6. The gene of claim 2 comprising the following substantially accurate DNA sequence:

| (5') | ATGTT | CAGCGAATTA | ATTAACTTCC |
|---|---|---|---|
| | AAAATGAAGG | TCATGAGTGC | CAATGCCAAT |
| | GTGGTAGCTG | CAAAAATAAT | GAACAATGCC |
| | AAAAATCATG | TAGCTGTCCA | ACGGGGTGTA |
| | ACAGCGACGA | CAAATGCCCT | TGCGGTAACA |
| | AGTCTGAAGA | AACCAAGAAG | TCATGCTGCT |
| | CTGGGAAATG | A | (3') |

7. An isolated gene expression unit comprising the Yeast Copper-Metallothionein gene promoter and the structural gene of claim 1 and mutants and genetically engineered derivatives thereof.

8. The gene expression unit of claim 7 which is present in a Sau3A-Sau3A fragment of chromosomal DNA in a copper-resistant (1 mM CuSO$_4$) mutant.

9. The gene expression unit of claim 8 which is present in a Sau3A-KpnI fragment of the chromosomal DNA.

10. The gene expression unit of claim 8 which is present in a DdeI-KpnI fragment of the chromosomal DNA.

11. The gene expression unit of claim 8 comprising the following substantially accurate DNA sequence:

(5')     TTAG     CCTTGTTACT     AGTTAGAAAA

AGACATTTTT     GCTGTCAGTC     ACTGTCAAGA

GATTCTTTTG     CTGGCATTTC     TTCTAGAAGC

AAAAAGAGCG     ATGCGTCTTT     TCCGCTGAAC

CGTTCCAGCA     AAAAAGACTA     CCAACGCAAT

ATGGATTGTC     AGAATCATAT     AAAAGAGAAG

CAAATAACTC     CTTGTCTTGT     ATCAATTGCA

TTATAATATC     TTCTTGTTAG     TGCAATATCA

TATAGAAGTC     ATCGAAATAG     ATATTAAGAA

AAACAAACTG     TACAATCAAT     CAATCAATCA

TCACATAAAA     TGTTCAGCGA     ATTAATTAAC

TTCCAAAATG     AAGGTCATGA     GTGCCAATGC

CAATGTGGTA     GCTGCAAAAA     TAATGAACAA

TGCCAAAAAT     CATGTAGCTG     TCCAACGGGG

TGTAACAGCG     ACGACAAATG     CCCTTGCGGT

AACAAGTCTG     AAGAAACCAA     GAAGTCATGC

TGCTCTGGGA     AATGA     (3')

12. An isolated gene promoter region comprising the Yeast Copper-Metallothionein promoter and mutants and genetically engineered derivatives thereof.

13. The promoter region of claim 12 which is present in a Sau3A-Sau3A fragment of chromosomal DNA in a copper-resistant (1 mM CuSO₄) mutant.

14. The promoter region of claim 13 which is present in a Sau3A-KpnI fragment of the chromosomal DNA.

15. The promoter region of claim 13 which is present in a Sau3A-RsaI fragment of the chromosomal DNA.

16. The promoter region of claim 13 which is present in a DdeI-RsaI fragment of the chromosomal DNA.

17. The promoter region of claim 13 comprising the following substantially accurate sequence:

| (5') | | |
|------|------|------|
| TTAG | CCTTGTTACT | AGTTAGAAAA |
| AGACATTTTT | GCTGTCAGTC | ACTGTCAAGA |
| GATTCTTTTG | CTGGCATTTC | TTCTAGAAGC |
| AAAAAGAGCG | ATGCGTCTTT | TCCGCTGAAC |
| CGTTCCAGCA | AAAAAGACTA | CCAACGCAAT |
| ATGGATTGTC | AGAATCATAT | AAAAGAGAAG |
| CAAATAACTC | CTTGTCTTGT | ATCAATTGCA |
| TTATAATATC | TTCTTGTTAG | TGCAATATCA |
| TATAGAAGTC | ATCGAAATAG | ATATTAAGAA |
| AAACAAACTG | | T    (3') |

18. The promoter region of claim 13 comprising the following substantially accurate sequence:

| (5') | | |
|------|------|------|
| TTAG | CCTTGTTACT | AGTTAGAAAA |
| AGACATTTTT | GCTGTCAGTC | ACTGTCAAGA |
| GATTCTTTTG | CTGGCATTTC | TTCTAGAAGC |
| AAAAAGAGCG | ATGCGTCTTT | TCCGCTGAAC |
| CGTTCCAGCA | AAAAAGACTA | CCAACGCAAT |
| ATGGATTGTC | AGAATCATAT | AAAAGAGAAG |
| CAAATAACTC | CTTGTCTTGT | ATCAATTGCA |
| TTATAATATC | TTCTTGTTAG | TGCAATATCA |
| TATAGAAGTC | ATCGAAATAG | ATATTAAGAA |

AAACAAACTG     TACAATCAAT     CAATCAATCA

TCACATAAA<u>A</u>     <u>TG</u>  (3')

19. A recombinant DNA molecule comprising the structural gene of any of claims 1 through 6.

20. A recombinant DNA molecule comprising the gene expression unit of any of claims 7 through 11.

21. A recombinant DNA molecule comprising the promoter region of any of claims 12 through 18.

22. The recombinant DNA molecule YEp1.

23. The recombinant DNA molecule YEp2.

24. The recombinant DNA molecule YEp29.

25. The recombinant DNA molecule YEp36.

26. The recombinant DNA molecule YRp20.

CLAIMS FOR THE CONTRACTING STATE: AT.

1. A method for producing yeast copper-metallothionein which comprises cloning on an expression vector in yeast or other eukaryotes or prokaryotes a structural gene derived from yeast which can code for Yeast Copper-Metallothionein, and mutants and genetically engineered derivatives thereof.

2. A method according to claim 1 wherein the gene is present in a Sau3A-Sau3A fragment of chromosomal DNA in a copper-resistant (1 mM $CuSO_4$) mutant.

3. A method according to claim 2 wherein the gene is present in a Sau3A-KpnI fragment of the chromosomal DNA.

4. A method according to claim 2 wherein the gene is present in a XbaI-KpnI fragment of the chromosal DNA.

5. A method according to claim 2 wherein the gene is present in a RsaI-KpnI fragment of the chromosal DNA.

6. A method according to claim 2 wherein the gene comprises the following substantially accurate DNA sequence:

```
(5')      ATGTT      CAGCGAATTA      ATTAACTTCC
          AAAATGAAGG  TCATGAGTGC      CAATGCCAAT
          GTGGTAGCTG  CAAAAATAAT      GAACAATGCC
          AAAAATCATG  TAGCTGTCCA      ACGGGGTGTA
          ACAGCGACGA  CAAATGCCCT      TGCGGTAACA
          AGTCTGAAGA  AACCAAGAAG      TCATGCTGCT
          CTGGGAAATG  A               (3')
```

-6-

0134773

7. A method for producing yeast cooper-metallothionein which comprises cloning on a vector in yeast a gene expression unit comprising the Yeast Copper-Metallothionein gene promoter and structural gene of claim 1 and mutants and genetically engineered derivatives thereof.

8. A method according to claim 7 wherein the gene expression unit is present in a Sau3A-Sau3A fragment of chromosomal DNA in a copper-resistant (1 mM CuSO$_4$) mutant.

9. A method according to claim 8 wherein the gene expression unit is present in a Sau3A-KpnI fragment of the chromosomal DNA.

10. A method according to claim 8 wherein the gene expression unit is 1 present in a DdeI-KpnI fragment of the chromosomal DNA.

11. A method according to claim 8 wherein the gene expression unit comprises the following substantially accurate DNA sequence:

| (5') | | | |
|---|---|---|---|
| | TTAG | CCTTGTTACT | AGTTAGAAAA |
| | AGACATTTTT | GCTGTCAGTC | ACTGTCAAGA |
| | GATTCTTTTG | CTGGCATTTC | TTCTAGAAGC |
| | AAAAAGAGCG | ATGCGTCTTT | TCCGCTGAAC |
| | CGTTCCAGCA | AAAAAGACTA | CCAACGCAAT |
| | ATGGATTGTC | AGAATCATAT | AAAAGAGAAG |
| | CAAATAACTC | CTTGTCTTGT | ATCAATTGCA |
| | TTATAATATC | TTCTTGTTAG | TGCAATATCA |

| | | |
|---|---|---|
| TATAGAAGTC | ATCGAAATAG | ATATTAAGAA |
| AAACAAACTG | TACAATCAAT | CAATCAATCA |
| TCACATAAAA | TGTTCAGCGA | ATTAATTAAC |
| TTCCAAAATG | AAGGTCATGA | GTGCCAATGC |
| CAATGTGGTA | GCTGCAAAAA | TAATGAACAA |
| TGCCAAAAAT | CATGTAGCTG | TCCAACGGGG |
| TGTAACAGCG | ACGACAAATG | CCCTTGCGGT |
| AACAAGTCTG | AAGAAACCAA | GAAGTCATGC |
| TGCTCTGGGA | AATGA (3') | |

12. A method for expressing a gene in yeast which comprises cloning in yeast on a plasmid upstream of the gene a gene promoter region comprising the Yeast Copper-Metallothionein promoter and mutants and genetically engineered derivatives thereof.

13. A method according to claim 12 wherein the promoter region is present in a Sau3A-Sau3A fragment of chromosomal DNA in a copper-resistant (1 mM CuSO$_4$) mutant.

14. A method according to claim 13 wherein the promoter region is present in a Sau3A-KpnI fragment of the chromosomal DNA.

15. A method according to claim 13 wherein the promoter region is present in a Sau3A-RsaI fragment of the chromosomal DNA.

16. A method according to claim 13 wherein the promoter region is present in a DdeI-RsaI fragment of the chromosomal DNA.

17. A method according to claim 13 wherein the promoter region comprises the following substantially accurate sequence:

```
(5')             TTAG      CCTTGTTACT     AGTTAGAAAA
            AGACATTTTT     GCTGTCAGTC     ACTGTCAAGA
            GATTCTTTTG     CTGGCATTTC     TTCTAGAAGC
            AAAAAGAGCG     ATGCGTCTTT     TCCGCTGAAC
            CGTTCCAGCA     AAAAAGACTA     CCAACGCAAT
            ATGGATTGTC     AGAATCATAT     AAAAGAGAAG
            CAAATAACTC     CTTGTCTTGT     ATCAATTGCA
            TTATAATATC     TTCTTGTTAG     TGCAATATCA

            TATAGAAGTC     ATCGAAATAG     ATATTAAGAA

            AAACAAACTG              T  (3')


            AAACAAACTG     TACAATCAAT     CAATCAATCA
            TCACATAAAA     TG      (3')
```

18. A method according to claim 13 wherein the promoter region comprises the following substantially accurate sequence:

```
(5')             TTAG      CCTTGTTACT     AGTTAGAAAA
            AGACATTTTT     GCTGTCAGTC     ACTGTCAAGA
            GATTCTTTTG     CTGGCATTTC     TTCTAGAAGC
            AAAAAGAGCG     ATGCGTCTTT     TCCGCTGAAC
            CGTTCCAGCA     AAAAAGACTA     CCAACGCAAT
            ATGGATTGTC     AGAATCATAT     AAAAGAGAAG
            CAAATAACTC     CTTGTCTTGT     ATCAATTGCA
            TTATAATATC     TTCTTGTTAG     TGCAATATCA
            TATAGAAGTC     ATCGAAATAG     ATATTAAGAA
```

19. A method for producing Yeast-Copper-Metallothionein which comprises cloning YEp1 in yeast

20. A method for producing Yeast-Copper-Metallothionein which comprises cloning YEp2 in yeast

21. A method for producing Yeast-Copper-Metallothionein which comprises cloning YEp29 in yeast

22. A method for producing Yeast-Copper-Metallothionein which comprises cloning YEp36 in yeast

23. A method for producing Yeast-Copper-Metallothionein which comprises cloning YRp20 in Yeast.

FIGURE 1

**European Patent Office**

**EUROPEAN SEARCH REPORT**

| DOCUMENTS CONSIDERED TO BE RELEVANT | | | EP 84870112.4 |
|---|---|---|---|
| Category | Citation of document with indication. where appropriate, of relevant passages | Relevant to claim | CLASSIFICATION OF THE APPLICATION (Int. Cl 4) |
| A,D | PROCEEDINGS OF THE NATIONAL ACADEMY OF SCIENCES OF THE UNITED STATES OF AMERICA, vol. 79, no. 17, September 1982, Baltimore, USA <br><br> S. FOGEL et al. "Tandem gene amplification mediates copper resistance in yeast" pages 5342-5346 <br><br> * Totality * <br><br>-- | 1-4,7-9,12-14,19-21 | C 12 N 15/00 |
| A | EP - A2 - 0 078 154 (THE REGENTS OF THE UNIVERSITY OF CALIFORNIA) <br><br> * Abstract; page 5, lines 32-38 * <br><br>---- | 1,7,12 | |
| | | | **TECHNICAL FIELDS SEARCHED (Int. Cl.4)** <br><br> C 12 N |

The present search report has been drawn up for all claims

| Place of search | Date of completion of the search | Examiner |
|---|---|---|
| VIENNA | 13-11-1984 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82